Europäisches Patentamt

European Patent Office

Office européen des brevets

(11) Veröffentlichungsnummer: **0 490 307 A2**

## (12) EUROPÄISCHE PATENTANMELDUNG

(21) Anmeldenummer: **91121032.6**

(22) Anmeldetag: **07.12.91**

(51) Int. Cl.5: **A61K 33/14**, A61M 1/16,
//A61M1/28,(A61K33/14,33:10,
31:22,31:70)

(30) Priorität: **11.12.90 DE 4039471**

(43) Veröffentlichungstag der Anmeldung:
**17.06.92 Patentblatt 92/25**

(84) Benannte Vertragsstaaten:
**AT BE CH DE DK ES FR GB IT LI NL**

(71) Anmelder: **B. Braun Melsungen AG**
**Carl-Braun Strasse**
**W-3508 Melsungen(DE)**

(72) Erfinder: **Meyer, Günther**
**Unterer Weinberg 5**
**W-3508 Melsungen 5(DE)**
Erfinder: **Stremetzne, Ralf**
**Friedrich-Ebert-Strasse 16**
**W-3582 Felsberg(DE)**
Erfinder: **Rath, Dieter**
**Franz-Gleim-Strasse 69**
**W-3508 Melsungen(DE)**

(74) Vertreter: **von Kreisler, Alek, Dipl.-Chem. et al**
**Patentanwälte Von Kreisler-Selting-Werner,**
**Deichmannhaus am Hauptbahnhof**
**W-5000 Köln 1(DE)**

(54) **Spezialkonzentrat für die Bicarbonat-Dialyse und seine Verwendung.**

(57) Die Erfindung betrifft eine Zusammensetzung für die BicarbonatDialyse, bestehend aus einem Behälter mit einem Grundkonzentrat, enthaltend $Na^+$, $K^+$, $Cl^-$, Acetat, Glucose sowie $HCO_3^-$ und einem Behälter mit einem Zusatzkonzentrat der als Kationen $Mg^{++}$ und $Ca^{++}$ enthält, sowie dessen Verwendung.

EP 0 490 307 A2

Die Erfindung betrifft eine Zusammensetzung für die Hämodialyse sowie ihre Verwendung.

Bicarbonat war ursprünglich die Puffersubstanz fur die Hämodialyse. Bicarbonat ersetzt direkt und ohne Umwege den natürlichen Puffer des Organismus. In der Pionierzeit der Hämodialyse war der technische Aufwand zur Bereitung von Bicarbonat-Dialysat jedoch enorm groß. Die umständliche und beschwerliche Prozedur wurde im Jahre 1964 aufgegeben, als automatische Proportionierungssysteme entwickelt waren.

In großen Mengen wurden dann flüssige Konzentrate benötigt. Bicarbonat kam wegen seiner Instabilität in Lösung und seiner begrenzten Lagerfähigkeit nicht länger als Puffer in Frage. Stattdessen erwies sich Acetat als ein äquivalentes Mittel für Dialyse-Zwecke. Seit etwa 1964 wurde Acetat der Standardpuffer des Dialysats. Acetat ist jedoch nur Bicarbonatersatz, da es in der Leber zu Bicarbonat umgewandelt wird. Vorteil des Acetats ist seine Stabilität in Lösung und damit seine unbegrenzte Lagerfähigkeit. Zudem ist es verhältnismäßig billig. Mit der Entwicklung hochwirksamer, großflächiger Dialysatoren konnte die Behandlungsdauer von Patienten erheblich verkürzt werden. In großflächigem Dialysator wird Bicarbonat sehr rasch aus dem Blut entfernt. Der Bicarbonatverlust und die Karenz, bis der Organismus Acetat in Bicarbonat umgewandelt hat, wird gegenwärtig für die Nebenwirkungen bei der Hämodialyse verantwortlich gemacht. Diese Problematik führte zu einer Rückbesinnung auf die Bicarbonat-Dialyse.

Die Bicarbonat-Dialyse wird heute bei folgenden Indikationen empfohlen:

Akutdialyse (Azidose), Kinderdialyse, Seniorendialyse, bei eingeschränkter Leberfunktion, bei mangelhafter Sauerstoffversorgung (Gewebshypoxie), bei Hyperkaliämie mit verminderter Auswurfleistung des Herzens.

Üblicherweise wird zur Zeit für die Bicarbonat-Dialyse eine Kombination von zwei Konzentraten verwendet, wobei eine sogenannte saure Komponente $Na^+$, $K^+$, $Ca^{++}$, $Mg^{++}$, $Cl^-$, Acetat sowie Glucose und eine zweite basische Komponente zumeist lediglich Bicarbonat, eventuell mit Spuren von $Na^+$, $Cl^-$ und Acetat, enthält. Anlageband zum Bundesgesetzblatt Teil I, Nr. 20 vom 19. März 1987: Anlage zur dritten Verordnung zur Änderung der Verordnung über Standardzulassungen vom 3. März 1987. Die Komponenten werden üblicherweise in je einem 10 l-Kanister aufbewahrt. Die Applikation erfolgt mittels einer Dosierungseinrichtung.

In Clinical Nephrology, 31, 132-138 (1989) wird ein Bicarbonat enthaltendes Konzentrat für den Einsatz in der Hämodialyse beschrieben. Dieses Konzentrat enthält $Na^+$, $Cl^-$, $Mg^{++}$ und Bicarbonat.

Die vorliegende Erfindung hat die Aufgabe, die Nachteile, die mit der im Stand der Technik verwendeten Zusammensetzung für die Bicarbonat-Dialyse auch verbunden sind, zu überwinden.

Die vorliegende Erfindung hat die Aufgabe, die Hämodialyse zu vereinfachen und damit Kosten und Material einzusparen. Dieses Ziel ist nicht zuletzt deshalb anzustreben, weil wegen des erheblichen Durchsatzes von Dialyseverbrauchsmaterialien jährlich beträchtliche Abfallmengen entstehen, die sowohl Kosten als auch ökologische Probleme nach sich ziehen.

Die Aufgabe wird gelöst durch eine Zusammensetzung für die Bicarbonat-Dialyse, bestehend aus einer Komponente, dem Grundkonzentrat, das $Na^+$ $K^+$, $Cl^-$, Acetat, Glucose sowie Bicarbonat enthält und einer zweiten Komponente, dem Zusatzkonzentrat, das als Kationen $Mg^{++}$ und $Ca^{++}$ enthält.

Die erfindungsgemäße Zusammensetzung besteht bevorzugt aus einem das Grundkonzentrat enthaltenden Behälter, der ein etwa zehnmal größeres Volumen besitzt als der Behälter mit dem Zusatzkonzentrat. Erfindungsgemäß vorteilhaft ist die Verwendung eines 10 l-Behälters für das Grundkonzentrat und eines 1 l-Behälters für das Zusatzkonzentrat.

Entsprechend der Erfindung soll ein Grundkonzentrat die Elektrolyte $Na^+$, $K^+$, $Cl^-$, Acetat sowie Glucose und Bicarbonat enthalten. Die Elektrolyte des Zusatzkonzentrats, $Ca^{++}$ und $Mg^{++}$, sollen in ein in der Infusionstechnik übliches Gebinde abgefüllt und per Infusionspumpe in den Dialysierflüssigkeitsbereich des Dialysegerätes eindosiert werden. Die erfindungsgemäße Zusammen-Setzung eignet sich zur Versorgung der auf dem Markt befindlichen Dialysegeräte.

Gegenüber den gebräuchlichen Zusammensetzungen für die Bicarbonat-Dialyse hat die erfindungsgemäße Zusammensetzung sehr entscheidende Vorteile.

1. Ein großer Vorteil der erfindungsgemäßen Zusammensetzung für die Bicarbonat-Dialyse ist die Erhöhung der Sicherheit für die Patienten. Falls etwa das Zusatzkonzentrat bei der Durchführung der Dialyse vergessen wird, fehlt dem Patienten kurzfristig lediglich $Ca^{++}$ und $Mg^{++}$. Bei den üblichen Zusammensetzungen für die Bicarbonatdialyse ist das Risiko bei der Verwendung am Patienten erheblich größer. Wird die Zuführung der basischen Komponente des Standes der Technik vergessen, fehlt dem Patienten Bicarbonat, was zu erheblichen Störungen führen kann, weil Bicarbonat als Puffersubstanz wirkt. Folge ist das Auftreten von Kopfschmerzen, Übelkeit, Erbrechen, Blutdruckabfall, Müdigkeit und Abgeschlagenheit sowie Muskelkrämpfe.

2. Bei den bekannten Zusammensetzungen für die Bicarbonat-Dialyse wird zwischen einer sauren und einer basischen Komponente unterschieden. Die Konzentrate weisen einen gravierenden Unterschied im Natriumgehalt auf. Während die sauren Komponenten einen sehr hohen Natriumgehalt von etwa 70 bis 130 mmol/l aufweisen, ist in den basischen Komponenten der $Na^+$-Gehalt sehr gering. Dies führt dazu, daß in den sauren Komponenten kein Wachstum unerwünschter Keime stattfindet; in den basischen Lösungen dagegen ist Keimwachstum möglich. Das Keimwachstum ist abhängig von der Keimzahl zu Produktionsbeginn und dem $Na^+$-Gehalt der Lösung, wobei das Keimwachstum sich zum $Na^+$-Gehalt umgekehrt proportional verhält.

Erfindungsgemäß liegt der $Na^+$-Gehalt deutlich über dem von im Stand der Technik benutzten Konzentraten. Das Risiko der Verkeimung innerhalb des Behälters, bezogen auf die Lagerzeit, ist also bei der erfindungsgemäßen Zusammensetzung wesentlich geringer.

Bei der Verwendung der erfindungsgemäßen Zusammensetzung kommt es zu Einsparungen von Verpackungsmaterial. Damit können die Herstellungskosten für die Zusammensetzung gesenkt werden. Die Applikation des Zusatzkonzentrats erfolgt erfindungsgemäß mittels einer Infusionspumpe, eine zusätzliche Dosierungseinrichtung für das basische Bicarbonat ist nicht notwendig. Im Ergebnis führt dies wiederum zum Senken der Kosten und gleichzeitig zum Senken der Abfallmengen.

Figur 1 zeigt die Verteilung der einzelnen Bestandteile der Konzentrate des Standes der Technik im Vergleich zu den erfindungsgemäßen Konzentraten.

3. Einer der beiden üblicherweise im Stand der Technik benötigten 10 l-Kanister für das basische bzw. saure Konzentrat kann durch einen wesentlich kleineren Kanister ersetzt werden. Gleichzeitig bietet die Verwendung eines kleineren Kanisters einen Vorteil unter dem Gesichtspunkt der Abfallvermeidung.

4. Bei der momentanen Form der Durchführung der Bicarbonatdialyse ergeben sich erhebliche Komplikationen, da das basische Bicarbonat als Puffersubstanz im Hauptkonzentrat fehlt.

Beispiel

Erfindungsgemäße Zusammensetzungen für die Bicarbonat-Dialyse bestehen aus einem Grundkonzentrat (Konzentrat I) und einem Zusatzkonzentrat (Konzentrat II) mit folgender Rezeptur:

| Konzentrat I | | Konzentrat II | |
|---|---|---|---|
| $Na^+$ | 138,0 mmol/l | $Ca^{++}$ | 1,75 mmol/l |
| $K^+$ | 3,0 mmol/l | $Mg^{++}$ | 0,5 mmol/l |
| $Cl^-$ | 110,5 mmol/l | | |
| Acetat | 3,0 mmol/l | | |
| Glucose | 1 g/l | | |
| Bicarbonat | 32,0 mmol/l | | |

Zusammensetzung der gebrauchsfertigen Hämodialyselösung nach folgendem Mischungsverhältnis:
1 l Konzentrat plus 34 l Wasser geeigneter Qualität

**Patentansprüche**

1. Zusammensetzung für die Bicarbonat-Dialyse, bestehend aus einem Behälter mit einem Grundkonzentrat, enthaltend $Na^+$, $K^+$, $Cl^-$, Acetat, Glucose sowie $HCO_3^-$ und einem Behälter mit einem Zusatzkonzentrat der als Kationen $Mg^{++}$ und $Ca^{++}$ enthält.

2. Zusammensetzung nach Anspruch 1, dadurch gekennzeichnet, daß der das Grundkonzentrat enthaltende Behälter ein etwa zehnmal größeres Volumen besitzt als der Behälter mit dem Zusatzkonzentrat.

3. Verwendung einer Zusammensetzung nach den Ansprüchen 1 und 2 für die Hämodialyse, dadurch gekennzeichnet, daß ein Zusatzkonzentrat, enthaltend $Ca^{2+}$ und $Mg^{2+}$ zusätzlich zu einem Grundkonzentrat, das $Na^+$, $K^+$, $Cl^-$, Acetat, Glucose sowie basisches Bicarbonat enthält, in ein in der Infusionstechnik übliches Gebinde abgefüllt wird und per Infusionspumpe in den Dialysierflüssigkeitsbereich eines Dialysegerätes eindosiert wird.